# EUROPEAN PATENT SPECIFICATION

(11) **EP 2 422 834 B1**
(45) Date of publication and mention of the grant of the patent: **06.09.2017**
(21) Application number: 10766995.4
(22) Date of filing: 15.04.2010
(51) Int. Cl.: A61M 21/02, A47C 3/025, A61H 23/02, A47C 7/72

(54) **RELAXATION DEVICE**
ENTSPANNUNGSVORRICHTUNG
DISPOSITIF DE RELAXATION

(30) Priority: 20.04.2009 JP 2009102028
(43) Date of publication of application: 29.02.2012
(73) Proprietor: Panasonic Corporation, Osaka 571-8501 (JP)
(72) Inventor: HAMATSUKA, Taichi, Kadoma-shi Osaka 571-8501 (JP); TSUTSUI, Shinji, Kadoma-shi Osaka 571-8501 (JP); TANIGUCHI, Shohei, Kadoma-shi Osaka 571-8501 (JP); YAMAMOTO, Kouhei, Kadoma-shi Osaka 571-8501 (JP); IKEBE, Munekiyo, Kadoma-shi Osaka 571-8501 (JP); NAKAMURA, Junji, Kadoma-shi Osaka 571-8501 (JP); NISHIO, Fumihiro, Kadoma-shi Osaka 571-8501 (JP); TANIZAWA, Takayoshi, Kadoma-shi Osaka 571-8501 (JP)
(74) Representative: Appelt, Christian W.
(86) International application number: PCT/JP2010/056742
(87) International publication number: WO 2010/122938

(56) References cited:
- EP-A1- 2 198 751
- CN-Y- 201 178 847
- JP-A- 2000 042 112
- JP-A- 2001 078 288
- JP-A- 2001 149 164
- JP-A- 2002 147 840
- JP-A- 2002 360 651
- JP-A- 2007 037 832
- JP-U- 3 017 242
- JP-U- H0 593 284
- JP-U- H02 119 840
- US-A- 4 038 499
- US-A- 5 645 578
- US-A- 5 838 808

## Description

### TECHNICAL FIELD

The present invention relates to a relaxation device that has a relaxation effect on a user.

### BACKGROUND ART

EP 2 198 751 A1 describes a relaxation apparatus including a body-supporting unit for supporting a user's body, a rocking unit for rocking the body-supporting unit at least in one direction and a control unit for controlling the rocking unit to rock the body-supporting unit. The relaxation apparatus further includes a manipulator by which a user selects any one of a plurality of courses in which amplitude and frequency of the rocking motion of the body-supporting unit are differently set.

US 5,645,578 describes a therapeutic device that provides light therapy in a sauna bed that has a hood that fits over the head of a person lying in the sauna bed. The hood can be pivoted from a retracted position to a usable position and is attached to a head end of the vibratory sauna bed. The hood provides a support for lights of selected hues or colors and bright, full spectrum, white light in the sunlight range that is positioned so that it will strike a person lying in the sauna bed in the same manner as normal, bright mid-day sunlight, and thereby provide for controllable light therapy. In addition to the light that directly shines on the head of a user, coordinated or corresponding light is also provided on the interior of the sauna bed compartment. The interior controllable light hue or color is identical to the hood light color to provide benefits of exposure of the body to colored lights. The therapeutic device can be utilized to provide for a controlled, highly integrated therapy arrangement using heat, vibration, aroma, sound and light.

JP 2001 149164 A describes a relaxation device comprising a human body supporting means, for example a chair with a backrest part and a seat part. The chair is driven by a drive means. The drive means is constituted of a base. Links have one end part pivoted at the base and the other end part pivoted at a lower part of a base part below the seat part of the chair. A motor is installed in a case. A drive device is provided for the motor. A power transmitting mechanism comprises a crank mechanism to transmit the power of the motor to the chair to rock the chair. A rod connects the power transmitting mechanism to the base part. The drive device is provided with a control means to change the rocking speed of the chair while the chair is reciprocated, and the relaxation device is capable of changing the rocking speed of the human body supporting means.

JP 2000 042112 A describes relaxing equipment comprising a support means such as a chair, and the chair is oscillated in a longitudinal direction by an exciting device. The exciting device applies rocking operations resulting in oscillations with a frequency of about 1 Hz to the chair. In order to avoid a monotonous feeling to a human body and achieve a reliable relaxation effect, the frequency is changed at every oscillation. The exciting device operates to change the frequency only gradually, wherein a fluctuation range is 0.2 Hz or less.

In the prior art, as described, for example, in patent document 1, a relaxation device includes a rocking driving means for rocking a body supporting means, which supports the body of the user, in forward and rearward directions, and a controlling means for controlling the rocking driving means to change the rocking amplitude of the body supporting means. The relaxation device has a relaxation effect on a user that is produced by changing the rocking amplitude.

### PRIOR ART DOCUMENT

Patent Document 1: Japanese Laid-Open Patent Publication No. 2000-300374

### DISCLOSURE OF THE INVENTION

In the relaxation device described above, the relaxation effect on the user is produced by rocking the body supporting means. However, the relaxation effect is low when only performing a rocking operation.

In order to satisfy such a demand, a speaker may be built into the body supporting means to produce a relaxation effect by generating sound (music) from the speaker. However, the sound generated from the speaker may be difficult to hear depending on where the speaker is installed.

Accordingly, it is an object of the present invention to provide a relaxation device that outputs the sound from the speaker so that the user can listen to the sound in a further effective manner and thereby produces a further desirable relaxation effect.

One embodiment of the present disclosure is a relaxation device. The relaxation device is provided with a body supporting means that supports a user's body and includes a seat and a backrest, which is arranged at a rear part of the seat. A rocking driving means rocks the body supporting means in at least one direction. A controlling means controls the rocking driving means. A speaker outputs sound. The speaker is arranged in the backrest of the body supporting means. The body supporting means includes a depression formed to be deeper at an inner side than an outer side of the body supporting means relative to a widthwise direction. A sound output direction of the speaker is tilted toward a center of the body supporting means in the widthwise direction.

In this structure, the sound from the speakers is transmitted in a satisfactory manner to the user. Thus, the user can listen to music in a further effective manner, and a further preferable relaxation effect is produced.

Preferably, the relaxation device further includes a cushion arranged in the backrest and containing a sponge member. The sponge member is formed so that density at a portion corresponding to the sound output direction of the speaker is lower than that at other portions of the cushion.

In this structure, the music (sound) from the speaker is transmitted in a satisfactory manner through the cushion. Thus, the user can listen to music in a further effective manner.

Preferably, the relaxation device further includes a pillow arranged on the backrest. A user's head can be placed on the pillow. The speaker is arranged at a position corresponding to the pillow in the backrest.

In this structure, the music from the speakers is effectively transmitted near the user's head.

Preferably, in the relaxation device, the pillow includes a rear surface, which has a protrusion formed with a shape conforming to the depression of the backrest, and a front surface, which is located opposite to the rear surface and formed to have a flat shape.

In this structure, the central portion of the pillow is thick, and the outer portion of the pillow is thin. Thus, the sound from the speaker arranged at the outer portion of the backrest is transmitted in a satisfactory manner.

Preferably, in the relaxation device, the pillow contains a sponge member, and the sponge member of the pillow is formed so that density at a portion corresponding to the sound output direction of the speaker is lower than other portions of the pillow.

In this structure, sound is transmitted from the speaker in a further effective manner, while maintaining satisfactory cushioning performance of the pillow.

Preferably, in the relaxation device, the backrest includes a cover serving as an outer surface of the backrest, and the cover includes a hole formed at a position corresponding to the sound output direction of the speaker.

In this structure, sound from the speaker is transmitted to the user through the hole in the cover to the user in a satisfactory manner.

Preferably, the relaxation device includes a vibration generator that generates vibration. The vibration generator is arranged in the body supporting means, and the controlling means controls the speaker and the vibration generator so that at least either one of sound and vibration is output in accordance with the rocking of the body supporting means.

In this structure, vibration or sound is output in cooperation with the rocking operation. This has a further effective relaxing effect on the user.

Preferably, the relaxation device is further provided with an operation unit including an operation switch, which sends an instruction to the controlling means when operated by the user, and a light, which indicates an operational state of the operation switch. The controlling means is configured to maintain the light in an illuminated state for a predetermined time when the operation unit is in an operable state, control the light to a non-illuminated state and shift the operation unit to a standby state after the predetermined time elapses, and switch the light from the non-illuminated state when the operation unit is touched to the illuminated state and shift the operation unit to the operable state.

In this structure, the user has to touch the operation unit once to operate the operation unit in the standby state and return the operation unit to the operable state. Accordingly, the settings of the rocking operation are not changed even when the user unintentionally touches the operation unit during the rocking operation. This prevents erroneous operation during the rocking operation.

Preferably, the relaxation device is further provided with an operation unit including an operation switch, which sends an instruction to the controlling means when operated by the user, and a light, which indicates an operational state of the operation switch. The controlling means is configured to maintain the light in an illuminated state for a predetermined time when the operation unit is in an operable state, and control the light to a flashing state and shift the operation unit to a standby state after the predetermined time elapses.

In this structure, the light flashes when the operation unit is in the standby state. Accordingly, the user can easily recognize that the operation unit is in the standby state. According to the invention, in the relaxation device, the controlling means sets a rocking speed at which the rocking of the body supporting means starts to a minimum speed of the rocking operation.

In this structure, the rocking speed at the beginning of the rocking operation when the user is not used to the rocking operation is slow. This prevents the so-called motion sickness and has an effective relaxation effect on the user.

Preferably, the controlling means sets the rocking speed to 0.3 Hz or lower during a period from when the rocking of the body supporting means starts to when at least 30 seconds elapses.

In this structure, the rocking speed at the beginning of the rocking operation when the user is not used to the rocking operation is slow. This prevents the so-called motion sickness and has an effective relaxation effect on the user.

Preferably, the relaxation device is further provided with an operation unit that sends an instruction to the controlling means when operated by the user. The operation unit includes a rocking operation adjustment switch that changes at least either one of speed and amplitude of the rocking operation of the body supporting means. The controlling means executes one or more rocking operation courses, each rocking operation course is set in advance to change a rocking operation content at a predetermined timing, and the controlling means changes the rocking operation content at the predetermined timing set in advance for the rocking operation course even when at least either one of the speed and amplitude of the rocking operation in each rocking operation course is changed by the rocking operation adjustment switch.

In this structure, the controlling means switches music or the like even when the speed or amplitude of the rocking operation is changed. Accordingly, the cooperation of the rocking operation and music is improved, and a further effective relaxing effect is produced for the user.

Preferably, in the relaxation device, the one or more rocking operation courses include a first rocking operation course that changes the rocking operation content from a first step to a second step when the predetermined timing is reached, and the controlling means repeats at least the first step twice or more until reaching the predetermined timing when the speed of the rocking operation in the first rocking operation course is increased by the rocking operation adjustment switch.

This structure prevents the second step from being started before reaching the predetermined timing.

Preferably, in the relaxation device, the controlling means skips at least the first step when reaching the predetermined timing if the speed of the rocking operation in the first rocking operation course is decreased by the rocking operation adjustment switch.

In this structure, the second step is immediately started as soon as the predetermined timing is reached.

### BRIEF DESCRIPTION OF THE DRAWINGS

Fig. 1 is a schematic diagram of a relaxation device according to one embodiment;
Fig. 2(a) is a perspective view of the relaxation device, and Fig. 2(b) is a cross-sectional view of a backrest;
Fig. 3 is a diagram showing the relationship of a cover of the backrest and a speaker;
Fig. 4 is a timing chart showing a rocking operation course;
Figs. 5(a), 5(b), and 5(c) are diagrams showing the material of a sponge member;
Fig. 6 is a diagram showing an operation unit; and
Fig. 7 is a block diagram showing the electrical configuration of the relaxation device.

### EMBODIMENTS OF THE INVENTION

One embodiment of the present invention will now be described with reference to the drawings.

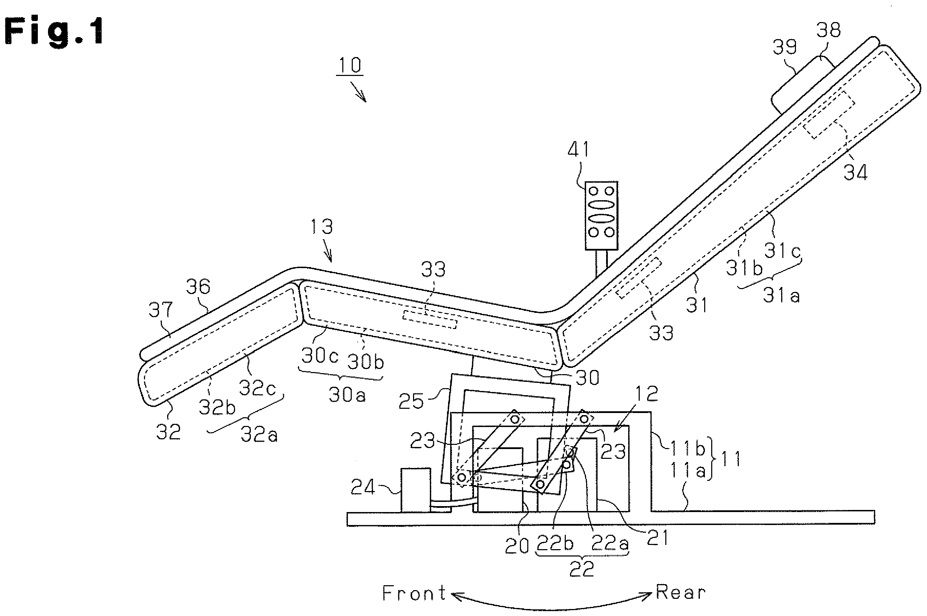

Fig. 1 shows a schematic diagram of a relaxation device of the present embodiment. As shown in Fig. 1, a relaxation device 10 includes a pedestal 11 with a bottom portion 11a placed on a floor surface (not shown), a rocking driver 12 serving as a rocking driving means arranged on the pedestal 11, and a body supporting member 13 serving as a body supporting means rocked by the rocking driver 12.

The rocking driver 12 includes a motor 20, a reduction gear 21, a crank mechanism 22, and link members 23 (e.g., two).

The motor 20 and a control unit 24, which serves as a controlling means for controlling and driving the motor 20, are arranged on an upper surface of the bottom portion 11a of the pedestal 11. The reduction gear 21 is also arranged on the upper surface of the bottom portion 11a of the pedestal 11. The reduction gear 21 is drive-coupled to the motor 20 to decelerate the driving speed of the motor 20. The motor 20 includes a position sensor (not shown) such as an encoder. The position sensor detects rotation speed and rotation position.

The crank mechanism 22, which includes a crank member 22a and a rod member 22b, converts the rotational drive of the reduction gear 21 to an arc motion having an extremely large radius. The crank member 22a includes a basal end, which is coupled to an output shaft of the reduction gear 21 so as to be rotatable integrally with the output shaft, and a distal end, which is coupled in a rotatable manner to a basal end of the rod member 22b. The rod member 22b includes a distal end connected to the lower part of a tetragonal frame-shaped base 25 to which the body supporting member 13 is fixed.

The pedestal 11 includes a support frame 11b projecting upward from the bottom portion 11a. Each of the two link members 23 includes a basal end, which is coupled in a rotatable manner to the upper part of the support frame 11b, and a distal end, which is coupled in a rotatable manner to the lower part of the base 25. The two link members 23 are spaced apart by a predetermined distance. The link members 23 are pivoted about their basal ends to rock the body supporting member 13, which is fixed to the base 25, in the forward and rearward directions like a rocking chair with the driving force from the crank mechanism 22.

The body supporting member 13 is chair-shaped. The body supporting member 13 includes a seat 30, which is fixed to the upper part of the base 25 and movable integrally with the base 25, a backrest 31, which is arranged at the rear part of the seat 30, and an ottoman 32, which is arranged at the front part of the seat 30. In the present embodiment, the backrest 31 and the ottoman 32 are each inclinable relative to the seat 30.

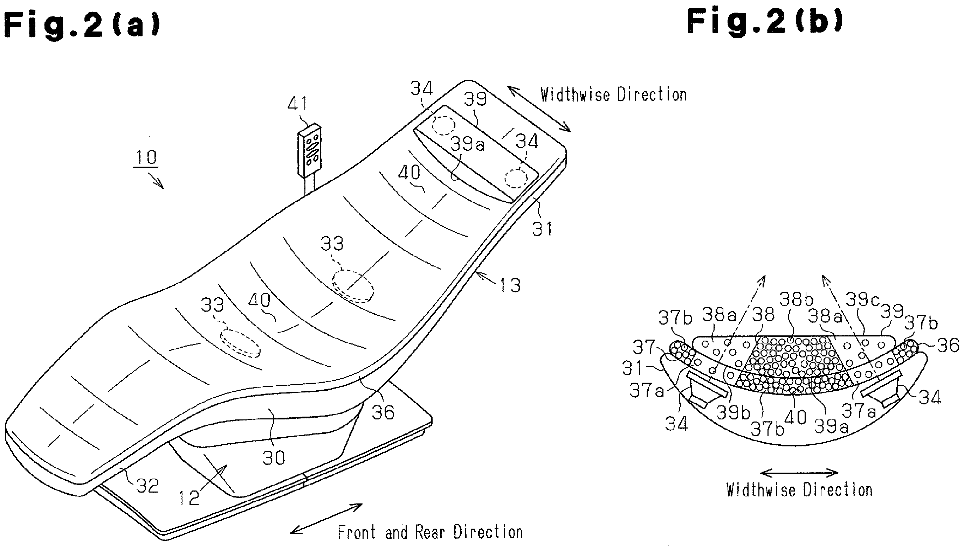

As shown in Figs. 2(a), 2(b), the body supporting member 13 includes a depression 40, which extends over the seat 30, the backrest 31, and the ottoman 32. The depression 40 has a smooth curved surface and is formed to be gradually deeper from the outer side toward the inner side (toward the center) in the widthwise direction of the body supporting member 13.
The depression 40 is continuous from the backrest 31 to the ottoman 32.

As shown in Fig. 1, the seat 30, the backrest 31, and the ottoman 32 respectively include a seat body 30a, a backrest body 31a, and an ottoman body 32a. The seat body 30a, the backrest body 31a, and the ottoman body 32a respectivefy include resin frames 30b, 31b, and 32b, and covers 30c, 31c, and 32c for the resin frames 30b, 31b, and 32b.

As shown in Fig. 1 and Fig. 2(a), the seat 30 and the backrest 31 each include a vibration transducer 33 serving as a vibration generator. A single vibration transducer 33 is arranged in each of the bodies 30a and 31a at a central position in the width direction. Thus, the relaxation device 10 can generate vibration.

As shown in Figs. 2(a), 2(b), two speakers 34 are arranged in the upper left and right sides of the backrest body 31a and tilted toward the center of the body supporting member 13 (backrest 31) relative to the widthwise direction. The sound generated from the speakers 34 is thus directed toward the center from the outer side in the widthwise direction of the body supporting member 13.

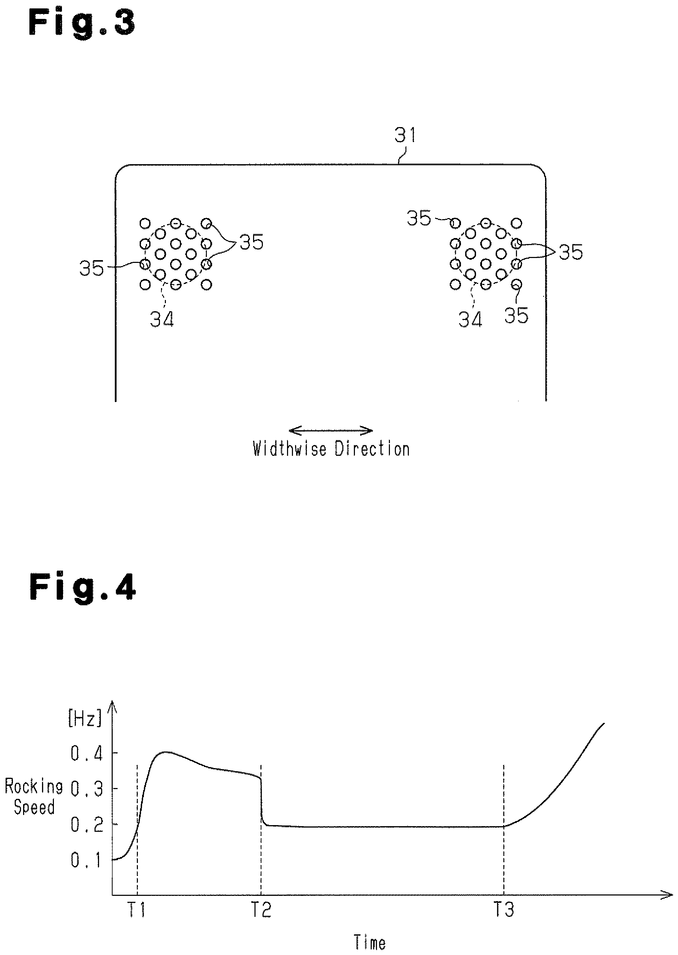

As shown in Fig. 3, the cover 31c of the backrest 31 includes holes 35 formed in portions corresponding to the sound output direction of the speakers 34, for example, portions overlapped with the speaker 34 as viewed from above. Thus, the sound from the speaker 34 is free of interference.

Further, as shown in Fig. 2(a), the surfaces of the covers 30c, 31c, and 32 are entirely covered by a cushion 36. The cushion 36 contains a sponge member 37 of, for example, urethane to soften impacts applied to a user's body. As shown in Fig. 2(b), the sponge member 37 is preferably formed by a low density member 37a and a high density member 37b, which has a higher density than the low density member 37a. In the present specification, the terms "high density" and "low density" simply represent relative density differences and do not limit the material of the sponge member. The low density member 37a is arranged at portions corresponding to the sound output direction (shown by single-dashed lines in Fig. 2(b)) of the speakers 34 in the backrest 31, for example, at positions overlapping the speakers 34 as view from above. The high density member 37b is arranged at the remaining portions.

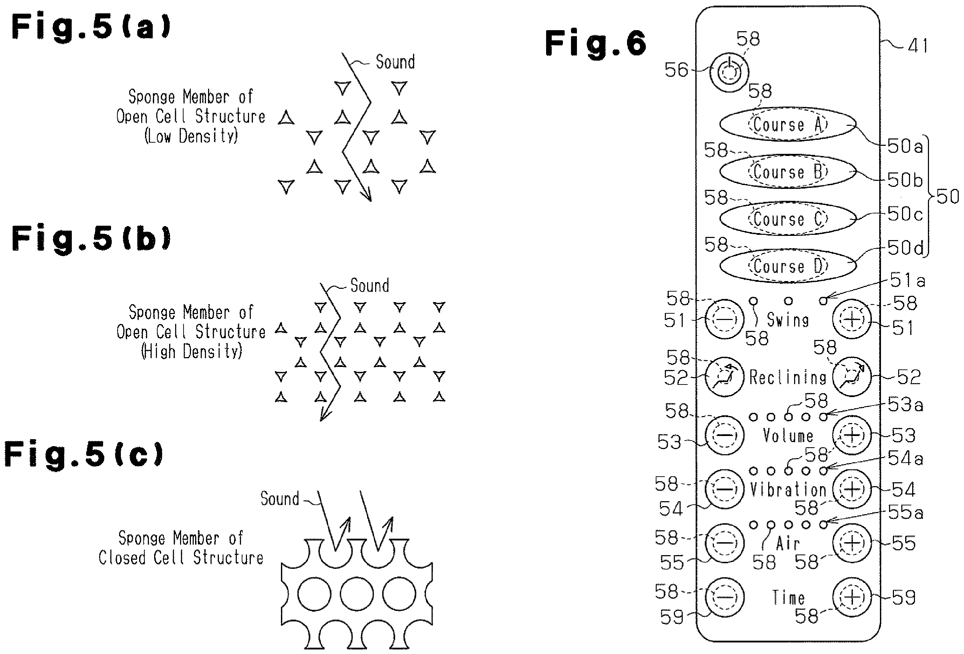

The sponge member 37 of the cushion 36 preferably has a so-called open cell structure. The open cell structure will be described below. As shown in Figs. 5(a) and 5(b), the open cell structure includes a plurality of pores in the sponge member 37. The pores are connected to each other. in contrast to the open cell structure, a closed cell structure, which is shown in Fig. 5(c), includes a plurality of pores in a sponge member that are independent from each other. That is, the pores are not connected. Thus, the sound (shown by solid arrow line in Fig. 5) from the speaker 34 is transmitted more efficiently through the interior of the sponge member 37 that has the open cell structure than a sponge member that has the closed cell structure. In particular, the number of pores is less in the low density sponge member 37 (37a) of Fig. 5(a) compared to the high density sponge member 37 (37b) of Fig. 5(b). Accordingly, the low density open cell structure efficiently transmits the sound from the speaker 34 as compared to the high density open cell structure.

Further, a pillow 39 containing a sponge member 38 of urethane or the like is arranged on an upper surface of the backrest 31 at the upper side (side farther from the seat 30). The pillow 39 supports the user's head. The pillow 39, which has a width that is slightly smaller than the width of the backrest 31, is arranged to cover the upper surface of the speaker 34.

The pillow 39 includes a rear surface 39a, on which a protrusion 39b is formed with a shape corresponding to the depression 40 of the body supporting member 13 (backrest 31), and a front surface 39c, which is flat. As shown in Figs. 2(a) and 2(b), the sponge member 38 of the pillow 39 is preferably formed by a low density member 38a and a high density member 38b, which has a higher density than the low density member 38a. The low density member 38a is arranged at portions corresponding to the sound output direction of the speaker 34 arranged in the backrest 31, for example, positions overlapping the speakers 34. The high density member 38b is arranged in the remaining portions. Accordingly, the sound from the speakers 34 is transmitted in a satisfactory manner even when the pillow 39 is arranged.

As shown in Fig. 1 and Fig. 2(a), an operation unit 41 is arranged on a side surface of the backrest 31 of the body supporting member 13 and supported by an operation unit holder (not shown) so that it can be attached to and detached from the holder.

As shown in Fig. 6, the operation unit 41 includes a plurality of operation switches such as course selection switches 50, swing adjustment switches 51, reclining angle adjustment switches 52, volume adjustment switches 53, vibration adjustment switches 54, airbag adjustment switches 55, and a start switch 56. The operation unit 41 includes lights 58 that are selectively switched between an illuminated state and a non-illuminated state to indicate the operational state of the operation switches 50 to 56.

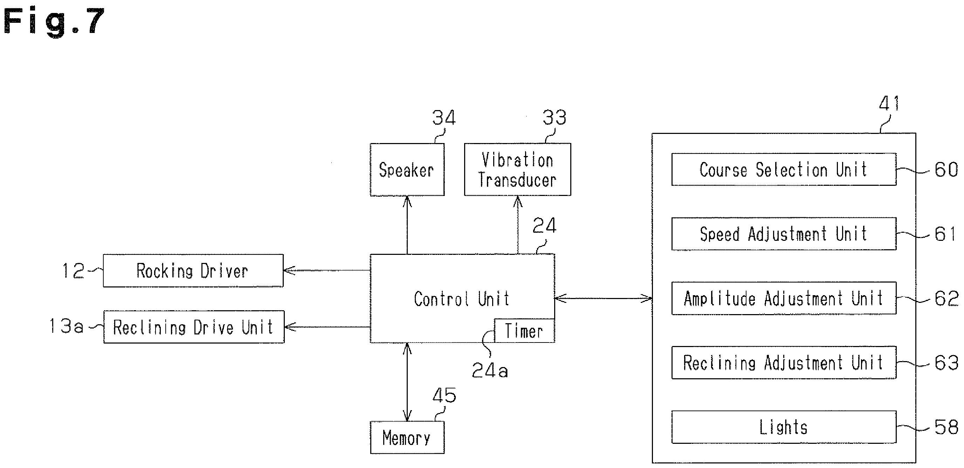

The course selection switches 50 includes a plurality of switches 50a, 50b, 50c, and 50d to select one of a plurality of courses (in the present embodiment, a total of four courses, which are course A, course B, course C, and course D) in which rocking patterns or the like are set. The rocking pattern of each course is set by the frequency and amplitude of a rocking operation in a course table (not shown) stored in a memory 45. When one of the switches 50a to 50d is selected, a predetermined control signal corresponding to the selected switch is output from a course selection unit 60, which is shown in Fig. 7, to the control unit 24.

The swing adjustment switches 51 are electrically connected to a speed adjustment unit 61 and an amplitude adjustment unit 62 shown in Fig. 7. The speed adjustment unit 61 adjusts the speed (frequency) of the rocking operation of the body supporting member 13, and the amplitude adjustment unit 62 adjusts the amplitude of the rocking operation. The swing adjustment switch 51 can change the speed (frequency) and the amplitude of the rocking operation in a stepped manner based on the operation of the user.

When the reclining angle adjustment switches 52 are operated by the user, a reclining adjustment unit 63, which is shown in Fig. 7, outputs a reclining adjustment control signal to the control unit 24. The control unit 24 controls a reclining drive unit 13a in response to the control signal and adjusts the reclining angle of the body supporting member 13.

The volume adjustment switches 53 adjust the volume of the speakers 34. The volume can be adjusted in five stages in the present embodiment. The vibration adjustment switches 54 adjusting the vibration degree of the vibration transducer 33 shown in Fig. 1 and Fig. 2. The vibration can be adjusted in five stages in the present embodiment. The airbag adjustment switches 55 change the inflation amount of the airbag (not shown). The inflation amount can be adjusted in five stages in the present embodiment.

The start switch 56 selectively switches the relaxation device 10 to a start enabled state or a start disabled state. Each unit can be controlled by the control unit 24 when the start switch 56 is operated and shifted to the start enabled state.

The lights 58 are formed by LEDs incorporated in a transparent section in a case of the operation unit 41. The lights 58 are provided for the switches 50 to 56. The lights 58 are also used as level indicators 51a, 53a, 54a, and 55a that indicate the present operational state of the switches 51, 53, 54, and 55. The lights 58 of the switches 51 to 55, which adjust the rocking operation and the like, are in a non-illuminated state when the operational state when the function corresponding to each switch is maximum or minimum. The control unit 24 continuously illuminates the lights 58 for a predetermined time (e.g., about five seconds) when the operation unit 41 is in an operable state. After the predetermined time (about five seconds) elapses, the control unit 24 controls the lights 58 to a non-illuminated state and shifts the operation unit 41 to a standby state. Further, when the operation unit 41 is touched in a non-illuminated state, the control unit 24 switches the lights 58 from the non-illuminated state to the illuminated state and shifts the operation unit 41 to the operable state.

The operation of the relaxation device 10 will now be described with reference to Figs. 1 to 5.

In the relaxation device 10, one of the plurality of (four in the present embodiment) courses, of which the rocking operation frequency or the like is set, is selected by operating a course selection switch 50 of the operation unit 41. The plurality of courses includes a course in which the rocking driver 12 is driven to perform relaxation rocking solely with the body supporting member 13, a course in which the relaxation rocking is performed in cooperation with the music generated from the speakers 34, and a relaxation rocking course implementing a refreshing function. In the relaxation rocking course implementing the refreshing function, "relaxation rocking" that has a relaxation effect on the user is first performed. Then, the actuation mode is switched from "relaxation rocking" to "refreshing rocking" that has a refreshing effect on the user.

In the present embodiment, the relaxation rocking course that performs the "relaxation rocking" and the "refreshing rocking" is set as, for example, "course A". "Course A" will now be described with reference to Fig. 4. Fig. 4 is a timing chart showing the rocking control that is executed when course A is started in a state in which none of the swing adjustment switches 51 of the operation unit 41 are pushed, that is, in a state in which the rocking operation speed is set to a standard value. The swing adjustment switches 51 can be operated to change the rocking speed in course A (the same applies to the other courses).

### [Course A]

When receiving a selection signal for course A from the operation unit 41, the control unit 24 controls the rocking speed (frequency) of the body supporting member 13 from 0.1 Hz, for example, and draws the user from an aroused state to a relaxed state. The control unit 24 controls the motor 20 at a rocking frequency of 0.3 Hz or lower until a predetermined time T1 (e.g., 30 seconds) elapses. When the rocking operation starts, the control unit 24 first always rocks the body supporting member 13 toward the front. After the predetermined time T1 elapses, the control unit 24 once raises the rocking speed to about 0.4 Hz. Then, the control unit 24 slows the rocking speed in steps until a predetermined time T2 (e.g., three minutes from when the rocking starts) elapses and performs the relaxation rocking at a rocking speed of 0.2 Hz. In this case, the control unit 24 selects predetermined music information I from internal music information stored in the memory 45 and outputs the music information I from the speakers 34 until the predetermined time T2 elapses. The control unit 24 also rocks the vibration transducer 33 in accordance with the sound of the music information 1.

From the predetermined time T2 to a predetermined time T3, the control unit 24 controls the rocking speed of the body supporting member 13 at 0.2 Hz, which is the lowest frequency (excluding that during activation of the frequency of course A and continues rocking to further relax the user and guide the user to a hypnotic state. During the period of predetermined times T2 to T3, the control unit 24 preferably outputs music information J, the tempo of which is slower than the music information I, from the speakers 34. In this manner, the control unit 24 selects music information corresponding to the rocking operation. Here, the control unit 24 further vibrates the vibration transducer 33 in accordance with the music information J.

After the predetermined time T3 elapses, the control unit 24 controls the motor 20 to perform refreshing rocking and guide the user from a rested state (relaxing state) to an aroused state. During refreshing rocking, the control unit 24 changes the rocking operation frequency in steps from 0.2 Hz to 0.4 Hz or greater (e.g., 0.6 Hz). Here, the control unit 24 preferably outputs music information K, the tempo of which is faster than the music information J and the pitch of which is higher than the music information I, from the speakers 34. The control unit 24 further vibrates the vibration transducer 33 in accordance with the sound of the music information K.

In this manner, in the relaxation device 10 of the present embodiment, the control unit 24 selects the internal music information stored in the memory 45 in accordance with each rocking course (or rocking operation) and applies vibration while generating music from the speakers 34, which serve as a generation means. Thus, the user is provided with sound (music) and vibration in accordance with the course. Accordingly, in addition to the relaxation effect produced by the conventional rocking, a further effective relaxation effect for the user is produced by the sound (music) and the vibration that are in accordance with the rocking operation.

Further, in the relaxation device 10 of the present embodiment, the user may operate the operation unit 41 to change the rocking speed (frequency) or amplitude in each of courses A to D from the standard value. In such a case, the control unit 24 switches, in an ensured manner, the operation of the rocking driver 12 to the next operation after a predetermined time elapses. The switching operation for [course A] will now be described (the same applies for other courses).

### [Switching Operation]

A plurality of steps performed in course A is set in a course table (not shown), which is stored in the memory 45. Course A is executed in order from the first step of the course table. Forward or rearward rocking is performed in each step. In the present example, a forward rocking operation is performed in an odd number step, and rearward rocking is performed in an even number step.

In the present example, when the rocking speed is set at the standard speed, that is, when the swing adjustment switches 51 is not pushed even once by the user before the rocking operation starts, the time schedule is set so that three minutes (predetermined time T2) elapses when the 50th step is performed. In other words, when the 51 st step starts, three minutes have elapsed. In this case, the rocking speed changes from 0.35 Hz to 0.2 Hz in the 51st step, as shown in Fig. 4.

For example, when the rocking operation starts in a state in which the rocking speed (frequency) is changed to become faster than the standard speed by the operation unit 41 (the swing adjustment switch 51 being pushed at least once), the 51 st step ends before the three minutes (predetermined time T2) elapses. In this case, the control unit 24 uses a timer 24a to measure the time from when the rocking starts. If three minutes have not elapsed when the 50th step ends, the control unit 24 repeats the 49th step and 50th step until three minutes elapses.

Further, for example, when the rocking operation starts in a state in which the rocking speed (frequency) is changed to become slower than the normal speed by the operation unit 41 (the swing adjustment switch 51 being pushed at least once), the 51 st step does not end before the three minutes (predetermined time T2) elapses. In this case, the control unit 24 uses the timer 24a to measure the time from when the rocking starts. If three minutes have elapsed, the control unit 24 skips the remaining steps and forcibly shifts to the 51st step. When skipping steps, it is preferable that an even number step be performed before shifting to the 51st step. This is because the 51st step is an odd number step in which the rocking is performed toward the front. Thus, by shifting to the 51st step after rocking toward the rear (even number step), the rocking operation can be smoothly performed.

By executing the control described above, shifting to the 51st step can be achieved in a relatively desirable manner without any discomfort even when the remaining steps are skipped. By performing the [switching operation] in such a manner, the time schedule of a course can be easily managed. As a result, the cooperation of the rocking operation and music is easily realized, and a further effective relaxation effect is produced.

In the present embodiment, the speaker 34 is arranged on the backrest 31 at opposite sides of the user's head, and the sound output direction is tilted toward the center of the backrest 31 relative to the widthwise direction (refer to Fig. 2(b)). Thus, when sound is output from the speakers 34 in each of courses A to D, the sound is transmitted in a satisfactory manner from the speakers 34. Further, the cushion 36 containing the sponge member 37 is arranged on the upper surface side of the backrest body 31a (cover 31c). The sponge member 37 of the cushion 36 is formed so that the density of the portion corresponding to the sound output direction of the speakers 34 (low density member 37a) is lower than the density of other portions (high density member 37b). Thus, music (sound) from the speakers 34 is transmitted in a satisfactory manner through the cushion 36, and the user can listen to music in a further effective manner.

In the backrest 31, the pillow 39 on which the head of the user can be placed is arranged on the cushion 36. The speakers 34 are arranged at a position corresponding to the pillow 39. Thus, the music from the speakers 34 is effectively transmitted at positions close to the user's head. The pillow 39 includes the rear surface 39a, on which the protrusion 39b is formed with a shape conforming to the depression 40 of the backrest 31, and the front surface 39c, which is formed to be flat. Accordingly, the pillow 39 at the central portion relative to the widthwise direction is thick and at the outer portion relative to the widthwise direction is thin. The sound from the speakers 34 arranged on the outer side relative to the widthwise direction of the backrest 31 is transmitted in a satisfactory manner, and satisfactory cushioning performance is obtained at the central portion of the pillow 39.

The pillow 39 contains the sponge member 38, and the sponge member 38 is formed so that the density at portion corresponding to the sound output direction of the speakers 34 (low density member 38a) is lower than the density at other portions (high density member 38b) of the pillow 39. Thus, the cushioning performance of the pillow 39 is maintained in a satisfactory manner, and the sound from the speakers 34 is effectively transmitted. The cover 31c that covers the resin frame 31b of the backrest body 31a includes the holes 35 formed at portions corresponding to the sound output direction of the speakers 34. This effectively prevents the sound from the speakers 34 from being interfered by the cover 31c.

When starting the rocking operation (e.g., when starting course A), the control unit 24 controls the rocking driver 12 to first rock toward the front. This prevents the user, who is supported in a relatively stable manner by the backrest 31, from feeling uncomfortable and produces an effective relaxation effect.

The relaxation device 10 of the present embodiment has the advantages described below.
(1) The body supporting member 13 includes the depression 40, of which the depth becomes deeper toward the inner side from the outer side relative to the widthwise direction of the body supporting member 13. The speakers 34 are arranged so that the sound output direction is tilted toward the center of the backrest 31 relative to the widthwise direction. Accordingly, the sound from the speakers 34 is transmitted in a satisfactory manner to the user, who is supported at a central position relative to the widthwise direction of the body supporting member 13. Thus, the user can listen to music in a further effective manner, and a further preferable relaxation effect is produced.
(2) The cushion 36 including the sponge member 37 is arranged on the upper surface side of the backrest 31 (body supporting member 13). The sponge member 37 includes the low density member 37a arranged at the position corresponding to the sound output direction of the speakers 34, and the high density member 37b is arranged at the remaining positions. Accordingly, the music (sound) from the speaker 34 is transmitted in a satisfactory manner through the cushion 36. Thus, the user can listen to music in a further effective manner.
(3) At the backrest 31, the pillow 39 on which the head of the user can be mounted is arranged on the upper surface of the cushion 36. The speakers 34 are arranged at positions corresponding to the pillow 39 of the backrest 31. Accordingly, the music from the speakers 34 is effectively transmitted near the user's head.
(4) The pillow 39 includes the front surface 39c, which is formed to be flat, and the rear surface 39a, on which the protrusion 39b is formed in conformance with the depression 40 of the backrest 31. Thus, in the pillow 39, the central portion is thick, and the outer portion is thin. Accordingly, the sound from the speakers 34 arranged at the outer portion of the backrest 31 is transmitted in a satisfactory manner. Further, the thick central portion of the pillow 39 obtains satisfactory cushioning performance of the pillow 39.
(5) The pillow 39 contains the sponge member 38, and the sponge member 38 includes the low density member 38a, which is arranged at positions corresponding to the sound output direction of the speakers 34, and the low density member 38b, which is arranged at the remaining positions. This transmits sound from the speakers 34 in a further effective manner, while maintaining satisfactory cushioning performance of the pillow 39.
(6) The cover 31c of the backrest body 31a includes the holes 35 formed at portions corresponding to the sound output direction of the speakers 34. Such a structure prevents the sound from the speakers 34 from being interfered by the cover 31c.
(7) The seat 30 and the backrest 31 of the body supporting member 13 each include the vibration transducer 33 serving as the vibration generator that generates vibration. The control unit 24 controls the speaker 34 and each vibration transducer 33 so that the sound output from the speakers 34 and the vibration generated by the vibration transducer 33 are both in accordance with the rocking operation. In this manner, by conforming vibration and music to the rocking operation, a cooperative feel is enhanced and a further effective relaxation effect is produced.
(8) The operation unit 41 includes the operation switches 50 to 56 that send instructions to the control unit 24 when operated by the user, and the lights 58 for indicate the operational state of the operation switches 50 to 56. When the operation unit 41 is in an operable state, the control unit 24 maintains the lights 58 in an illuminated state for a predetermined time. Further, after the predetermined time elapses, the control unit 24 controls the lights 58 to a non-illuminated state and shifts the operation unit 41 to the standby state. When the operation unit 41 is touched in the non-illuminated state, the control unit 24 switches the lights 58 from the non-illuminated state to the illuminated state and shifts the operation unit 41 to the operable state. That is, the operation unit 41 needs to be touched once to operate the operation unit 41 in the standby state. Accordingly, the settings of the rocking operation are not changed even when the user unintentionally touches the operation unit 41 during the rocking operation. This prevents erroneous operation during the rocking operation and improves the safety of the apparatus.
(9) The control unit 24 controls the rocking driver 12 so that the rocking speed when the rocking operation starts is the slowest (0.1 Hz). In this manner, the rocking speed at the beginning of the rocking operation when the user is not used to the rocking operation is slow. This prevents the so-called motion sickness and has an effective relaxation effect on the user.
(10) The control unit 24 controls the rocking driver 12 so that the rocking speed becomes lower than or equal to 0.3 Hz during the period of at least 30 seconds from the start of the vibration. Thus, occurrence of so-called motion sickness is suppressed and effective relaxation effect is produced on the user by slowing the rocking speed at the start of the vibration when the user is not used to the vibration.
(11) The control unit 24 is capable of performing one or more rocking operation courses (e.g., course A). Each rocking operation course is set in advance so that the rocking operation contents are changed at a predetermined timing. The swing adjustment switches 51, which serve as rocking operation adjustment switches, are arranged on the operation unit 41 to change at least either one of the speed and amplitude of the rocking operation. Even when at least either one of the speed and amplitude of the rocking operation in each rocking operation course is changed by the swing adjustment switches 51, the control unit 24 controls the rocking driver 12 so that the rocking operation contents are changed at a predetermined timing that is set in advance for the rocking operation course. Accordingly, even when the rocking speed is changed from the standard speed, the control unit 24 manages the time schedule of the course to ensure that the operation changes at the predetermined timing. Thus, the control unit 24 appropriately switches music in accordance with the rocking operation. This improves the cooperation of the rocking operation and music, and a further effective relaxation effect is produced.
(12) The control unit 24 controls the rocking driver 12 so that the rocking operation is first performed in the forward direction when the rocking operation starts. Such a structure prevents the user, who is supported in a relatively stable manner by the backrest 31, from feeling uncomfortable and produces an effective relaxation effect.

The embodiment of the present invention may be modified as described below.

The sponge member 37 of the cushion 36 may be formed with the same density irrespective of the sound output direction of the speakers 34. In the same manner, the sponge member 38 of the pillow 39 may be formed with the same density irrespective of the sound output direction of the speakers 34. That is, the sponge members 37 and 38 may each be formed entirely by a low density member or entirely by a high density member. When the sponge members 37 and 38 are formed by low density members, the sound from the speakers 34 can be transmitted to the user in the same manner as the embodiment described above.

In each of courses A to D, the rocking speed when the rocking operation starts does not have to be the lowest during the course.

In each of course A to D, the rocking speed does not have to be 0.3 Hz or lower during the period until of 30 seconds elapses from when the rocking operation starts. Further, this period does not have to be fixed to 30 seconds. For example, this period may be changed in accordance with the difference in the course time of each course. Alternatively, this period may be set by the user in any manner. This allows for application to individual differences between users.

The arrangement of the speakers 34 is not limited to the upper side (side of the backrest 31 opposite to the seat 30) of the backrest 31 and may be arranged at the lower side of the backrest 31.

The pillow 39 does not have to cover the speaker 34.

The rear surface 39a of the pillow 39 does not have to be formed as the protrusion 39b and may have other shapes such as a depressed shape or a flat shape.

The front surface 39c of the pillow 39 does not have to have a flat shape and may have other shapes such as a depressed shape or a protruding shape.

The pillow 39 may be eliminated.

The operation unit 41 for operating the control unit 24 is not essential.

The standby state of the operation unit 41 does not have to be indicated by the non-illuminated state of the lights 58. For example, the control unit 24 may control the lights 58 to switch the non-illuminated state and the illuminated state in the standby state of the operation unit 41. That is, the standby state of the operation unit 41 may be indicated by flashing the lights 58. In this case, the user can also easily recognize the standby state of the operation unit 41.

In the embodiment described above, the control unit 24 adjusts the vibration from the vibration transducer 33 serving as a vibration generator in accordance with the music generated from the speakers 34 to indirectly adjust the vibration of the vibration transducer 33 in accordance with the rocking of the body supporting member 13. However, the vibration does not have to be adjusted in accordance with the music from the speakers 34.

In the embodiment described above, the vibration generator does not have to be formed by the vibration transducer 33. For example, the vibration generator may be formed by a so-called vibrator that generates vibration by rotating a motor shaft with a weight attached thereto that offsets the center of gravity. The vibration generator may also be eliminated.

The cover 31c of the backrest 31 does not have to include the holes 35.

In the embodiment described above, the music is output from the speakers 34 using the music information stored in the memory 45 in advance. instead, an external sound source may be used with an external input terminal arranged in the operation unit 41 or the like.

In the embodiment described above, the control unit 24 switches to the next operation at a predetermined timing by sequentially executing the steps of the course table (not shown) and repeating or skipping steps when the rocking speed is changed. However, the present invention is not limited in such a manner. For example, a time adjustment switch 59 may be arranged on the operation unit 41, and the predetermined timing for switching operations may be adjusted by the time adjustment switch 59. In such a structure, the rocking operation is performed in accordance with the preferred time of the user.

At least one of the ottoman 32 and the backrest 31 may be non-inclinable.

The body supporting member 13 does hat have to include the ottoman 32.

The body supporting member 13 may be rocked in the left and right directions in addition to in the front and rear directions.

## Claims

1. A relaxation device (10) comprising:
a body supporting means (13) that is configured to support a user's body and includes a seat (30) and a backrest (31), which is arranged at a rear part of the seat (30);
a rocking driving means (12) for rocking the body supporting means (13) in at least front and rear directions;
a controlling means (24) for controlling the rocking driving means (12) to perform a rocking operation of the body supporting means (13); and
a speaker (34) that is configured to output sound, wherein the speaker (34) is arranged in the backrest (31) of the body supporting means (13);
wherein the body supporting means (13) includes a depression (40) formed to be deeper at an inner side than an outer side of the body supporting means (13) relative to a widthwise direction; and
a sound output direction of the speaker (34) is tilted toward a center of the body supporting means (13) in the widthwise direction;
the relaxation device **characterized in that**:
the controlling means (24) is configured to control the rocking driving means (12) so that the controlling means (24) first always rocks the body supporting means (13) toward the front when the rocking operation of the body supporting means (13) starts, and sets a rocking speed at which the rocking of the body supporting means (13) starts to a minimum speed of the rocking operation.

2. The relaxation device (10) according to claim 1, **characterized by** further comprising a cushion (36) arranged in the backrest (31) and containing a sponge member (37), wherein the sponge member (37) is formed so that density at a portion corresponding to the sound output direction of the speaker (34) is lower than that at other portions of the cushion (36).

3. The relaxation device (10) according to claim 1 or 2, **characterized by** further comprising a pillow (39) arranged on the backrest (31), wherein a user's head can be placed on the pillow (39) and the speaker (34) is arranged at a position corresponding to the pillow (39) in the backrest (31).

4. The relaxation device (10) according to claim 3, **characterized in that** the pillow (39) includes:
a rear surface (39a) having a protrusion (39b) formed with a shape conforming to the depression (40) of the backrest (31); and
a front surface (39c) located opposite to the rear surface (39a) and formed to have a flat shape.

5. The relaxation device (10) according to claim 3 or 4, **characterized in that**:
the pillow (39) contains a sponge member (38); and
the sponge member (38) of the pillow (39) is formed so that density at a portion corresponding to the sound output direction (34) of the speaker is lower than that at other portions of the pillow (39).

6. The relaxation device (10) according to any one of claims 1 to 5, **characterized in that** the backrest (31) includes a cover (31c) serving as an outer surface of the backrest (31), and the cover (31c) includes a hole (35) formed at a position corresponding to the sound output direction of the speaker (34).

7. The relaxation device (10) according to any one of claims 1 to 6, **characterized by** further comprising a vibration generator (33) that generates vibration, wherein the vibration generator (33) is arranged in the body supporting means (13), and the controlling means (24) controls the speaker (34) and the vibration generator (33) so that at least either one of sound and vibration is output in accordance with the rocking operation of the body supporting means (13).

8. The relaxation device (10) according to any one of claims 1 to 7, **characterized by** further comprising an operation unit (41) including an operation switch (50-56), which is configured to send an instruction to the controlling means (24) when operated by the user, and a light (58), which is configured to indicate an operational state of the operation switch (50-56);
wherein the controlling means (24) is configured to maintain the light (58) in an illuminated state for a predetermined time when the operation unit (41) is in an operable state, control the light (58) to a non-illuminated state and shift the operation unit (41) to a standby state after the predetermined time elapses, and switch the light (58) from the non-illuminated state to the illuminated state when the operation unit (41) is touched and shift the operation unit (41) to the operable state.

9. The relaxation device (10) according to any one of claims 1 to 7, **characterized by** further comprising an operation unit (41) including an operation switch (50-56), which is configured' to send an instruction to the controlling means (24) when operated by the user, and a light (58), which is configured to indicate an operational state of the operation switch (50-56);
wherein the controlling means (24) is configured to maintain the light (58) in an illuminated state for a predetermined time when the operation unit (41) is in an operable state, and control the light (58) to a flashing state and shift the operation unit (41) to a standby state after the predetermined time elapses.

10. The relaxation device (10) according to any one of claims 1 to 9, **characterized in that** the controlling means (24) is configured to set the rocking speed to 0.3 Hz or lower during a period from when the rocking of the body supporting means (13) starts to when at least 30 seconds elapses.

11. The relaxation device (10) according to any one of claims 1 to 10, **characterized by** further comprising an operation unit (41) that is configured to send an instruction to the controlling means (24) when operated by the user, wherein the operation unit (41) includes a rocking operation adjustment switch that is configured to change at least either one of speed and amplitude of the rocking operation of the body supporting means (13);
wherein the controlling means (24) is capable of performing one or more rocking operation courses, each rocking operation course is set in advance to change a rocking operation content at a predetermined timing, and the controlling means (24) is configured to change the rocking operation content at the predetermined timing set in advance for the corresponding rocking operation course even when at least either one of the speed and amplitude of the rocking operation in the corresponding rocking operation course is changed by the rocking operation adjustment switch (51).

12. The relaxation device (10) according to claim 11, **characterized in that**:
the one or more rocking operation courses include a first rocking operation course that changes the rocking operation content from a first step to a second step when the predetermined timing is reached; and
the controlling means (24) is configured to repeat at least the first step twice or more until the predetermined timing is reached when the speed of the rocking operation in the first rocking operation course is increased by the rocking operation adjustment switch (51).

13. The relaxation device (10) according to claim 12, **characterized in that** the controlling means (24) is configured to skip at least the first step when the predetermined timing is reached if the speed of the rocking operation in the first rocking operation course is decreased by the rocking operation adjustment switch (51).

## Patentansprüche

1. Entspannungseinrichtung (10), die umfasst:
ein Körperstützmittel (13), das derart ausgestaltet ist, dass es einen Körper eines Benutzers stützt und einen Sitz (30) und eine Rückenlehne (31) aufweist, die an einem hinteren Teil des Sitzes (30) angeordnet ist;
ein Wipp-Antriebsmittel (12) zum Wippen mit dem Körperstützmittel (13) in mindestens eine Vorwärts- und eine Rückwärtsrichtung;
ein Steuermittel (24) zum Steuern des Wipp-Antriebsmittels (12), um einen Wippvorgang des Körperstützmittels (13) durchzuführen; und
einen Lautsprecher (34) der derart ausgestaltet ist, dass er Ton ausgibt, wobei der Lautsprecher (34) in der Rückenlehne (31) des Körperstützmittels (13) angeordnet ist;
wobei das Körperstützmittel (13) eine Vertiefung (40) aufweist, die derart ausgebildet ist, dass sie relativ zu einer Breitenrichtung tiefer an einer Innenseite als an einer Außenseite des Körperstützmittels (13) ist; und
eine Tonausgaberichtung des Lautsprechers (34) in der Breitenrichtung zu einer Mitte des Körperstützmittels (13) hin geneigt ist;
wobei die Entspannungseinrichtung **dadurch**
**gekennzeichnet ist, dass**:
das Steuermittel (24) derart ausgestaltet ist, dass es den Wipp-Antriebsmechanismus (12) derart steuert, dass das Steuermittel (24) als Erstes das
Körperstützmittel (13) immer zu der Vorderseite hin wippen lässt, wenn der Wippvorgang des Körperstützmittels (13) beginnt, und eine Wippgeschwindigkeit, mit der das Wippen des Körperstützmittels (13) beginnt, auf eine Minimalgeschwindigkeit des Wippvorgangs einstellt.

2. Entspannungseinrichtung (10) nach Anspruch 1, **dadurch gekennzeichnet, dass** sie ferner ein Polster (36) umfasst, das in der Rückenlehne (31) angeordnet ist und ein Schwammelement (37) enthält, wobei das Schwammelement (37) derart ausgebildet ist, dass eine Dichte an einem Abschnitt, der der Tonausgaberichtung des Lautsprechers (34) entspricht, niedriger als an anderen Abschnitten des Polsters (36) ist.

3. Entspannungseinrichtung (10) nach Anspruch 1 oder 2, **dadurch gekennzeichnet, dass** sie ferner ein Kissen (39) umfasst, das auf der Rückenlehne (31) angeordnet ist, wobei der Kopf eines Benutzers auf dem Kissen (39) platziert werden kann, und der Lautsprecher (34) an einer Position angeordnet ist, die dem Kissen (39) in der Rückenlehne (31) entspricht.

4. Entspannungseinrichtung (10) nach Anspruch 3, **dadurch gekennzeichnet, dass** das Kissen (39) aufweist:
eine hintere Fläche (39a) mit einem Vorsprung (39b), der in einer Form ausgebildet ist, die mit der Vertiefung (40) der Rückenlehne (31) übereinstimmt; und
eine vordere Fläche (39c), die entgegengesetzt zu der hinteren Fläche (39a) liegt und derart ausgebildet ist, dass sie eine ebene Form aufweist.

5. Entspannungseinrichtung (10) nach Anspruch 3 oder 4, **dadurch gekennzeichnet, dass**:
das Kissen (39) ein Schwammelement (38) aufweist; und
das Schwammelement (38) des Kissens (39) derart ausgebildet ist, dass eine Dichte an einem Abschnitt, der der Tonausgaberichtung (34) des Lautsprechers entspricht, niedriger als an anderen Abschnitten des Kissens (39) ist.

6. Entspannungseinrichtung (10) nach einem der Ansprüche 1 bis 5, **dadurch gekennzeichnet, dass** die Rückenlehne (31) einen Bezug (31c) aufweist, der als eine Außenfläche der Rückenlehne (31) dient, und der Bezug (31c) eine Öffnung (35) aufweist, die an einer Position ausgebildet ist, die der Tonausgaberichtung des Lautsprechers (34) entspricht.

7. Entspannungseinrichtung (10) nach einem der Ansprüche 1 bis 6, **dadurch gekennzeichnet, dass** sie ferner einen Vibrationserzeuger (33) umfasst, der Vibration erzeugt, wobei der Vibrationserzeuger (33) in dem Körperstützmittel (13) angeordnet ist, und das Steuermittel (24) den Lautsprecher (34) und den Vibrationserzeuger (33) derart steuert, dass Klang und/oder Vibration im Einklang mit dem Wippvorgang des Körperstützmittels (13) ausgegeben werden.

8. Entspannungseinrichtung (10) nach einem der Ansprüche 1 bis 7, **dadurch gekennzeichnet, dass** sie ferner eine Betriebseinheit (41), die einen Betriebsschalter (50 bis 56) aufweist, der derart ausgestaltet ist, dass er eine Anweisung an das Steuermittel (24) sendet, wenn er von dem Benutzer betätigt wird, und eine Leuchte (58) umfasst, die derart ausgestaltet ist, dass sie einen Betriebszustand des Betriebsschalters (50 bis 56) anzeigt;
wobei das Steuermittel (24) derart ausgestaltet ist, dass es die Leuchte (58) über eine vorgegebene Zeit in einem beleuchteten Zustand hält, wenn sich die Betriebseinheit (41) in einem betriebsfähigen Zustand befindet, die Leuchte (58) derart steuert, dass sie in einen nicht beleuchteten Zustand versetzt wird, und die Betriebseinheit (41) in einen Bereitschaftszustand umschaltet, nachdem die vorgegebene Zeit verstrichen ist, und die Leuchte (58) aus dem nicht beleuchteten Zustand in den beleuchteten Zustand schaltet, wenn die Betriebseinheit (41) berührt wird, und die Betriebseinheit (41) in den betriebsfähigen Zustand umschaltet.

9. Entspannungseinrichtung (10) nach einem der Ansprüche 1 bis 7, **dadurch gekennzeichnet, dass** sie ferner eine Betriebseinheit (41), die einen Betriebsschalter (50 bis 56) aufweist, der derart ausgestaltet ist, dass er eine Anweisung an das Steuermittel (24) sendet, wenn er von dem Benutzer betätigt wird, und eine Leuchte (58) umfasst, die derart ausgestaltet ist, dass sie einen Betriebszustand des Betriebsschalters (50 bis 56) anzeigt;
wobei das Steuermittel (24) derart ausgestaltet ist, dass es die Leuchte (58) über eine vorgegebene Zeit in einem beleuchteten Zustand hält, wenn sich die Betriebseinheit (41) in einem betriebsfähigen Zustand befindet, und die Leuchte (58) derart steuert, dass sie in einen blinkenden Zustand versetzt wird, und die Betriebseinheit (41) in einen Bereitschaftszustand umschaltet, nachdem die vorgegebene Zeit verstrichen ist.

10. Entspannungseinrichtung (10) nach einem der Ansprüche 1. bis 9, **dadurch gekennzeichnet, dass** das Steuermittel (24) derart ausgestaltet ist, dass es während einer Zeitspanne ab dem Beginn des Wippens des Körperstützmittels (13) bis zu einem Zeitpunkt, zu dem mindestens 30 Sekunden verstrichen sind, die Wippgeschwindigkeit auf 0,3 Hz oder darunter einstellt.

11. Entspannungseinrichtung (10) nach einem der Ansprüche 1 bis 10, **dadurch gekennzeichnet, dass** sie ferner eine Betriebseinheit (41) umfasst, die **dadurch gekennzeichnet ist, dass** sie eine Anweisung an das Steuermittel (24) sendet, wenn sie von dem Benutzer betätigt wird, wobei die Betriebseinheit (41) einen Wippvorgangs-Einstellschalter aufweist, der derart ausgestaltet ist, dass er Geschwindigkeit und/oder Amplitude des Wippvorgangs des Körperstützmittels (13) ändert;
wobei das Steuermittel (24) in der Lage ist, einen oder mehrere Wippvorgangsverläufe durchzuführen, wobei jeder Wippvorgangsverlauf im Voraus eingestellt wird, um einen Wippvorgangsinhalt zu einem vorgegebenen Zeitpunkt zu ändern, und das Steuermittel (24) derart ausgestaltet ist, dass es den Wippvorgangsinhalt zu dem im Voraus für den entsprechenden Wippvorgangsverlauf eingestellten vorgegebenen Zeitpunkt ändert, selbst wenn die Geschwindigkeit und/oder die Amplitude des Wippvorgangs bei dem entsprechenden Wippvorgangsverlauf mithilfe des Wippvorgangs-Einstellschalters (51) geändert wird.

12. Entspannungseinrichtung (10) nach Anspruch 11, **dadurch gekennzeichnet, dass**:
zu dem einen oder mehreren Wippvorgangsverläufen ein erster Wippvorgangsverlauf zählt, der den Wippvorgangsinhalt von einem ersten Schritt zu einem zweiten Schritt ändert, wenn der vorgegebene Zeitpunkt erreicht ist; und
das Steuermittel (24) derart ausgestaltet ist, dass es mindestens den ersten Schritt zweimal oder öfter wiederholt, bis der vorgegebene Zeitpunkt erreicht ist, wenn die Geschwindigkeit des Wippvorgangs bei dem ersten Wippvorgangsverlauf mithilfe des Wippvorgangs-Einstellschalters (51) erhöht wird.

13. Entspannungseinrichtung (10) nach Anspruch 12, **dadurch gekennzeichnet, dass** das Steuermittel (24) derart ausgestaltet ist, dass es mindestens den ersten Schritt überspringt, wenn der vorgegebene Zeitpunkt erreicht ist, wenn die Geschwindigkeit des Wippvorgangs bei dem ersten Wippvorgangsverlauf mithilfe des Wippvorgangs-Einstellschalters (51) verringert wird.

## Revendications

1. Dispositif de relaxation (10) comprenant :
un moyen de support de corps (13) qui est configuré pour supporter le corps d'un utilisateur et comprend un siège (30) et un dossier (31), qui est agencé au niveau d'une partie arrière du siège (30) ;
un moyen d'entraînement de balancement (12) pour balancer le moyen de support de corps (13) dans au moins les directions avant et arrière ;
un moyen de commande (24) pour commander le moyen d'entraînement de balancement (12) afin de réaliser une opération de balancement du moyen de support de corps (13) ; et
un haut-parleur (34) qui est configuré pour produire du son, dans lequel le haut-parleur (34) est agencé dans le dossier (31) du moyen de support de corps (13) ;
dans lequel le moyen de support de corps (13) comprend un enfoncement (40) formé pour être plus profond d'un côté interne que d'un côté externe du moyen de support de corps (13) par rapport à une direction dans le sens de la largeur ; et
une direction de sortie de son du haut-parleur (34) est inclinée vers un centre du moyen de support de corps (13) dans la direction du sens de la largeur ;
le dispositif de relaxation étant **caractérisé en ce que** :
le moyen de commande (24) est configuré pour commander le moyen d'entraînement de balancement (12) de sorte que le moyen de commande (24) balance toujours en premier lieu le moyen de support de corps (13) vers l'avant lorsque l'opération de balancement du moyen de support de corps (13) commence, et détermine une vitesse de balancement à laquelle le balancement du moyen de support de corps (13) commence, sur une vitesse minimum de l'opération de balancement.

2. Dispositif de relaxation (10) selon la revendication 1, **caractérisé en ce qu'**il comprend en outre un coussin (36) agencé dans le dossier (31) et contenant un élément d'éponge (37), dans lequel l'élément d'éponge (37) est formé de sorte que la densité au niveau d'une partie correspondant à la direction de sortie de son du haut-parleur (34) est inférieure à celle au niveau des autres parties du coussin (36).

3. Dispositif de relaxation (10) selon la revendication 1 ou 2, **caractérisé en ce qu'**il comprend un coussin (39) agencé sur le dossier (31), dans lequel la tête d'un utilisateur peut être placée sur le coussin (39) et le haut-parleur (34) est agencé dans une position correspondant au coussin (39) dans le dossier (31).

4. Dispositif de relaxation (10) selon la revendication 3, **caractérisé en ce que** le coussin (39) comprend :
une surface arrière (39a) ayant une saillie (39b) formée avec une forme se conformant à l'enfoncement (40) du dossier (31) ; et
une surface avant (39c) opposée à la surface arrière (39a) et formée pour avoir une forme plate.

5. Dispositif de relaxation (10) selon la revendication 3 ou 4, **caractérisé en ce que** :
le coussin (39) contient un élément d'éponge (38) ; et
l'élément d'éponge (38) du coussin (39) est formé de sorte que la densité au niveau d'une partie correspondant à la direction de sortie de son (34) du haut-parleur est inférieure à celle au niveau des autres parties du coussin (39).

6. Dispositif de relaxation (10) selon l'une quelconque des revendications 1 à 5, **caractérisé en ce que** le dossier (31) comprend un revêtement (31c) servant de surface externe du dossier (31), et le revêtement (31c) comprend un trou (35) formé dans une position correspondant à la direction de sortie de son du haut-parleur (34).

7. Dispositif de relaxation (10) selon l'une quelconque des revendications 1 à 6, **caractérisé en ce qu'**il comprend en outre un générateur de vibration (33) qui génère une vibration, dans lequel le générateur de vibration (33) est agencé dans le moyen de support de corps (13) et le moyen de commande (24) commande le haut-parleur (34) et le générateur de vibration (33) de sorte qu'au moins l'un parmi le son et la vibration est produit selon l'opération de balancement du moyen de support de corps (13).

8. Dispositif de relaxation (10) selon l'une quelconque des revendications 1 à 7, **caractérisé en ce qu'**il comprend en outre une unité d'actionnement (41) comprenant un commutateur d'actionnement (50-56), qui est configuré pour envoyer une instruction au moyen de commande (24) lorsqu'il est actionné par l'utilisateur, et une lumière (58) qui est configurée pour indiquer un état opérationnel du commutateur d'actionnement (50-56) ;
dans lequel le moyen de commande (24) est configuré pour maintenir la lumière (58) dans un état allumé pendant une période de temps prédéterminée lorsque l'unité d'actionnement (41) est dans un état pouvant être actionné, commander la lumière (58) dans un état non allumé et faire passer l'unité d'actionnement (41) dans un état d'attente après l'écoulement de la période de temps prédéterminée, et faire passer la lumière (58) de l'état non allumé à l'état allumé lorsque l'unité d'actionnement (41) est touchée et faire passer l'unité d'actionnement (41) à l'état opérationnel.

9. Dispositif de relaxation (10) selon l'une quelconque des revendications 1 à 7, **caractérisé en ce qu'**il comprend en outre une unité d'actionnement (41) comprenant un commutateur d'actionnement (50-56) qui est configuré pour envoyer une instruction au moyen de commande (24) lorsqu'il est actionné par l'utilisateur, et une lumière (58) qui est configurée pour indiquer un état opérationnel du commutateur d'actionnement (50-56) ;
dans lequel le moyen de commande (24) est configuré pour maintenir la lumière (58) dans un état allumé pendant une période de temps prédéterminée lorsque l'unité d'actionnement (41) est dans un état pouvant être actionné, et commander la lumière (58) dans un état clignotant et faire passer l'unité d'actionnement (41) dans un état d'attente après l'écoulement de la période de temps prédéterminée.

10. Dispositif de relaxation (10) selon l'une quelconque des revendications 1 à 9, **caractérisé en ce que** le moyen de commande (24) est configuré pour régler la vitesse de balancement sur 0,3 Hz ou moins pendant une période à partir du moment où le balancement du moyen de support de corps (13) commence jusqu'au moment où au moins 30 secondes se sont écoulé.

11. Dispositif de relaxation (10) selon l'une quelconque des revendications 1 à 10, **caractérisé en ce qu'**il comprend en outre une unité d'actionnement (41) qui est configurée pour envoyer une instruction au moyen de commande (24) lorsqu'elle est actionnée par l'utilisateur, dans lequel l'unité d'actionnement (41) comprend un commutateur d'ajustement d'opération de balancement qui est configuré pour changer au moins l'une ou l'autre parmi la vitesse et l'amplitude de l'opération de balancement du moyen de support de corps (13) ;
dans lequel le moyen de commande (24) est capable de réaliser une ou plusieurs courses opérationnelles de balancement, chaque course opérationnelle de balancement est déterminée à l'avance pour changer un contenu d'opération de balancement à un moment prédéterminé, et le moyen de commande (24) est configuré pour modifier le contenu d'opération de balancement au moment prédéterminé déterminé à l'avance pour la course d'opération de balancement correspondante même lorsque au moins l'une ou l'autre parmi la vitesse et l'amplitude de l'opération de balancement dans la course opérationnelle de balancement correspondante est modifiée par le commutateur d'ajustement d'opération de balancement (51).

12. Dispositif de relaxation (10) selon la revendication 11, **caractérisé en ce que** :
les une ou plusieurs courses opérationnelles de balancement comprennent une première course opérationnelle de balancement qui change le contenu d'opération de balancement d'une première étape à une seconde étape lorsque le temps prédéterminé est atteint ; et
le moyen de commande (24) est configuré pour répéter au moins la première étape deux fois ou plus jusqu'à ce que le temps prédéterminé soit atteint lorsque la vitesse de l'opération de balancement dans la première course opérationnelle de balancement est augmentée par le commutateur d'ajustement d'opération de balancement (51).

13. Dispositif de relaxation (10) selon la revendication 12, **caractérisé en ce que** le moyen de commande (24) est configuré pour sauter au moins la première étape lorsque le temps prédéterminé est atteint, si la vitesse de l'opération de balancement dans la première course opérationnelle de balancement est diminuée par le commutateur d'ajustement d'opération de balancement (51).
